# EUROPÄISCHE PATENTSCHRIFT

(11) **EP 1 075 551 B1**
(45) Veröffentlichungstag und Bekanntmachung des Hinweises auf die Patenterteilung: **19.12.2001**
(21) Anmeldenummer: 99948556.8
(22) Anmeldetag: 03.05.1999
(51) Int. Cl.: C12Q 1/68

(54) **VERFAHREN ZUR NICHT-RADIOAKTIVEN DETEKTION VON MEMBRAN-GEBUNDENEN NUKLEINSÄUREN UND TESTKIT**
METHOD FOR NON-RADIOACTIVE DETECTION OF MEMBRANE-BONDED NUCLEIC ACIDS AND TEST KIT
PROCEDE POUR LA DETECTION NON RADIOACTIVE D'ACIDES NUCLEIQUES LIES A UNE MEMBRANE, ET MATERIEL DE TEST

(30) Priorität: 06.05.1998 DE 19821116; 07.12.1998 DE 19856391
(43) Veröffentlichungstag der Anmeldung: 14.02.2001
(73) Patentinhaber: Genprofile AG, 13125 Berlin (DE)
(72) Erfinder: HOEHE, Margret, D-10557 Berlin (DE); DELBRÜCK, Sebastian, D-10437 Berlin (DE)
(74) Vertreter: Baumbach, Friedrich, Dr.
(86) Internationale Anmeldenummer: DE9901066
(87) Internationale Veröffentlichungsnummer: WO9957307

(56) Entgegenhaltungen:
- EP-A- 0 806 431
- US-A- 5 605 795
- RICHTERICH P UND CHURCH G M: "DNA sequencing with direct transfer electrophoresis and nonradioactive detection" METHODS IN ENZYMOLOGY, Bd. 218, Nr. 1, - 1993 XP002114585 in der Anmeldung erwähnt
- DELBRÜCK S UND HOEHE M R: "Effektiver Einsatz von Chemilumineszenz bei der Generierung von Sequenzdaten" BIOSPEKTRUM, Bd. 2, 1998, Seiten 75-76, XP002114586
- HUGHES J R ET AL.: "Non-isotopic detection of nucleic acids on membranes. In: Non-Isotopic Methods in Molecular Biology" 1995 , IRL PRESS , OXFORD, UK XP002114588 Seite 145 - Seite 147 Seite 166 - Seite 175
- RAYMUNDO A K ET AL.: "Modification of a non-radioactive method for a membrane-based nucleic acid detection" PHILIPPINE JOURNAL OF SCIENCE, Bd. 123, Nr. 3, 1994, Seiten 281-288, XP002114587

## Beschreibung

Die vorliegende Erfindung betrifft ein neues Verfahren zur nicht-radioaktiven Detektion von Membran-gebundenen Nukleinsäuren, worunter Nukleinsäuren, die z. B. 'Single Nucleotide Polymorphisms' (SNP's) enthalten, DNA-Arrays (Cosmide, Yeast artificial chromosomes [YACs], Bacterial artificial chromosomes [BACs], cDNAs, PCR-Fragmente, Oligonukleotide), RNA-Arrays und jedwede Nukleinsäurefragmente, die aus Gelen (Agarose oder PAA) auf Membranen übertragen wurden, darunter genomische DNA-/Plasmid-DNA-Fragmente (Southern) und mRNAs (Northern), zu verstehen sind, sowie einen Testkit zur Durchführung des Verfahrens.

Arrays sind in einem definierten Maß vorzugsweise auf einer Membran gerastert angeordnete Proben einzelner Nukleinsäuren.

Die Identifizierung von Austauschen einzelner Basen innerhalb der DNA (SNP's) ist für die Erforschung der Ursachen komplexer Erkrankungen, z.B. von Bluthochdruck sowie für diagnostische Zwecke von großer Bedeutung *(Lander, E.S. (1996):* Science *274, 536-539, Collins, F.S. et al. (1997) Science 278, 1580-1581, Marshall, E. (1997): Science 277, 1752-1753).* Durch Sequenzierung der interessierenden Bereiche können diese Austausche erkannt werden.

Herkömmlicherweise wird die Sequenz durch das enzymatische Kettenabbruchverfahren bestimmt, in dem die Sequenzierungsprodukte durch den Einbau von radioaktiven oder nicht-radioaktiv markierten Nukleotiden markiert werden. Im Anschluß an die Sequenzierung und die gelelektrophoretische Auftrennung kann die Sequenz durch die Auswertung der Sequenzgele ermittelt werden. Die direkte Markierung der Sequenzierungsprodukte hat jedoch den Nachteil, daß nur ein einziges DNA-Fragment pro Sequenzierungsansatz bearbeitet werden kann.

Die Multiplex-Technik (siehe EP-A 0 303 459) überkommt diesen Nachteil durch die simultane Sequenzierung mehrerer DNA-Moleküle. Die entstandenen Gemische von Sequenzierungsprodukten werden gelelektrophoretisch aufgetrennt, auf eine Nylonmembran übertragen und dort fixiert. Durch Hybridisierung dieser Membran mit jeweils für ein einziges DNA-Fragment spezifischen Sonde können sukzessive auf der gleichen Membran die Sequenzen mehrerer DNA-Fragmente gelesen werden. Ein großer Nachteil dieser Methode besteht jedoch darin, daß die Detektion der unterschiedlichen DNA-Fragmente durch die Verwendung von radioaktiv markierten Sonden geschieht. Eine Automatisierung des gesamten Prozesses zur weiteren Steigerung des Durchsatzes ist deshalb nicht durchführbar.

Weiterhin bekannt ist ein nicht-radioaktives Detektionsverfahren, das von Richterich und Church (*Richterich, P., Church, G.M. (1993) Methods Enzymol. 218: 187-222)* beschrieben wurde, jedoch die Nachteile besitzt, nicht für sukzessive Hybridisierungen geeignet zu sein und die Hybridisierungen herkömmlicherweise nicht ausschließlich bei Raumtemperatur durchgeführt werden.

S. Dehlbrück & M. R. Hoehe (1998) Biospektrum 2, 75-76; US-A-5,605,795 und EP-A-0 806 431 offenbaren Verfahren zum Nachweis von DNA Sequenzen mittels Chemoluminiszenz. J. R. Hughes et al. (1995) in Non-isotopic Methods in Molecular Biology, S. 145-182, IRL Press, Oxford (UK) und P. Richterich & G. M. Church (1993) Methods in Enzymology 218, 187-222 offenbaren eine Reihe von routinemässigen Protokollen zum nicht-isotopischen Nachweis von Membran-gebundenen Nukleinsäuren. Keines der genannten Dokumente offenbart ein Verfahren, bei dem die Hybridisierungsreaktion um Raumtemperatur durchgeführt werden kann.

Aufgabe der vorliegenden Erfindung ist es deshalb, ein einfach durchzuführendes nicht-radioaktives Verfahren zu etablieren, daß zum einen die Sensitivität von radioaktiven Verfahren beibehält oder gar verbessert und zum anderen eine sofortige, direkte sukzessive Detektion ermöglicht.

Die Erfindung wird gemäß den Ansprüchen realisiert. Überraschend kann diese Aufgabe durch ein Verfahren gelöst werden, das in allen Schritten bei Raumtemperatur stattfinden kann. Das erfindungsgemäße Verfahren ist zur nicht-radioaktiven Detektion von Membran-gebundenen Nukleinsäuren (NA) geeignet, in dem man eine nach an sich bekannten Verfahren erhaltene NA, gebunden an eine Membran, mit einem speziellen Hybridisierungspuffer hybridisiert, der Tris-HCl, Tris-Base, NaCl, Triton X-100, SDS und Block Reagenz enthält, und anschließend die gebundenen NA detektiert. Nicht zum Umfang der Erfindung gehört die Detektion von Sequenzierungsprodukten nach dem Multiplex-Verfahren nach EP 303 459 (G. Church).

Vorzugsweise wird Membran-gebundene DNA detektiert.

Das Verfahren ist bevorzugt durch die folgenden Schritte gekennzeichnet:
1. Vorhybridisierung einer nach an sich bekannten Verfahren erhaltenen DNA enthaltenden Membran vorzugsweise in einer Schale, mit einem speziellen Hybridisierungspuffer,
2. Hybridisierung der Membran mit einer 5'-biotinylierten Sonde in dem gleichen Puffer,
3. mehrmaliges Waschen der Membran mit Puffer I, der PBS (Na₂HPO₄, NaH₂PO₄, NaCl, pH ca. 7,3), SDS und Block Reagenz enthält,
4. Inkubation der Membran mit Streptavidin-Alkalische-Phosphatase-Konjugat in Puffer I,
5. einmaliges Waschen der Membran mit Puffer I, danach mehrmaliges Waschen mit Puffer II, der PBS und SDS enthält, sowie mehrmaliges Äquilibrieren bei pH 9,75 mit Puffer III, der Tris-HCl und Diethanolamin enthält,
6. nach der Äquilibrierung Überführung der Membran in eine Substratlösung, die sich aus CDP-Star (Tropix) und Puffer III zusammensetzt, und ggf. mehrmaliges Aufnehmen und Ablegen der Membran zwecks gleichmäßiger Verteilung der Substratlösung,
7. nach kurzem Abtropfen Fixierung der Membran, z.B. auf einem Stück Plastik, und Abdeckung, bevorzugt mit einer Klarsichtfolie,
8. exponieren, bevorzugt mit einem Röntgenfilm oder einer CCD-Kamera,
9. nach Exposition, Rückführung der Membran und mehrmaliges Strippen mit vorgewärmtem Puffer IV, der EDTA und SDS enthält,
10.einmaliges Waschen mit Puffer V, der Tris-HCl und NaCl enthält.

Die Auswertung erfolgt nach an sich üblichen Methoden, wie z.B. dem Prinzip der 'skilled pattern analysis'.

Der wichtigste Schritt im o.g. Verfahren ist die Hybridisierung mit einem speziellen Hybridisierungspuffer, wodurch mehrfach aufeinanderfolgende Hybridisierungen möglich werden und eine sensitivität erreicht wird, die der radioaktiven Detektion gleicht bzw. teilweise verbessert ist. Damit stellt das erfindungsgemäße Verfahren zur nicht-radioaktiven Detektion von Nukleinsäuren, die z. B. 'Single Nucleotide Polymorphisms' (SNP's) enthalten, einen großen Fortschritt dar. Darüber hinaus kann der gesundheitsschädigende Einsatz von Radioaktivität vermieden werden. Außerdem konnten durch Zusammenfassung ansonsten zeitaufwendiger Zwischenschritte sowie durch Optimierung der Zusammensetzungen der Lösungen und der notwendigen Waschparameter gleichbleibende Ergebnisse erreicht werden, wodurch die Basis für die zukünftige Entwicklung eines automatisierten Detektionsautomaten geschaffen werden konnte.

Der erfindungsgemäße Testkit zur nicht-radioaktiven Detektion von Nukleinsäuren, die z.B. 'Single Nucleotide Polymorphisms' (SNP's) enthalten, enthält die folgenden Lösungen:
Hybridisierungspuffer : 5 l
   500 ml 10xTNT
   1250 ml 20% SDS
   10g Block Reagenz (Boehringer Mannheim, DIG-Kit)
   3250 ml H₂O (bidest)
10xPBS: 5 l
   516.2 g Na₂HPO₄
   132.6 g NaH₂PO₄
   198.7 g NaCl
   H₂O ad 5l pH: ca. 7.3
Puffer I: 5 l
   10 g Block Reagenz
   250 ml 10xPBS
   125 ml 20% SDS
   4625 ml H₂O (bidest), in der Microwelle 8 min erhitzen, rühren bis Block Reagenz gelöst ist
Puffer II: 5 l
   250 ml 10xPBS
   125 ml 20% SDS
   4625 ml H₂O (bidest)
10xPuffer III: 2 l
   64.4 g TrisHCl
   1770 ml H₂O (bidest)
   210.4 g Diethanolamin pH: 9.75 (einstellen!)
Puffer IV: 5 l
   4700 ml H₂O (bidest)
   50 ml 0.5M EDTA, pH8.0
   250 ml 20% SDS
Puffer V: 1 l
   920 ml H₂O (bidest)
   30 ml 5M NaCl
   50 ml 1M TrisCl, pH8.0
10xTNT: 1 l
   44.4 g TrisHCl
   25.6 g TrisBase
   73 g NaCl
   818 ml H₂O (bidest)
   100 ml TritonX-100 pH: ca. 8.0

### Ausführungsbeispiel

Alle Schritte werden bei Raumtemperatur durchgeführt.
1. Membran mit der DNA-Seite nach oben in einer Schale mit 180 ml Hybridisierungspuffer (28 mM Tris-HCl / 21 mM TrisBase / 125 mM NaCl / 1% Triton X-100 / 5% SDS / 0.2% Block Reagenz) für 1h vorhybridisieren. Rocky: 12°/0.5
2. Vorhybridisierungslösung verwerfen. 5'-biotinylierte Sonde für 5 min. bei max. Umdrehung zentrifugieren und sofort in einer Endkonzentration von 10 pmol/ml zu 100 ml Hybridisierungspuffer geben (200µM/5µl). Membran für 1h hybridisieren. Rocky: 12°/0.5
3. Hybridisierungslösung vollständig entfernen. Membran 5x 5 min mit 120 ml Puffer I (29 mM Na₂HPO₄ / 8 mM NaH₂PO₄ / 34 mM NaCl / 0.5% SDS, 0.2% Block Reagenz) waschen. Waschlösung immer vollständig entfernen. Darauf achten, daß sich keine großen Luftblasen unterhalb der Membran befinden. Rocky: 12°/10 zum Einfüllen der Lösung, danach langsam auf 12°/6 zum Waschen runterregulieren.
4. waschlösung vollständig entfernen. Membran 10 min mit 4 µl Streptavidin-Alkalische-Phosphatase-Konjugat (vor Zugabe 5 min bei max. Umdrehung zentrifugieren, Boehringer Ingelheim) in 100 ml Puffer I inkubieren. Rocky: 12°/0.5.
5. SvAP-Lösung vollständig entfernen. Membran 1x 5 min mit 120 ml Puffer I und danach 6 x 5 min mit 120 ml Puffer II (29 mM Na₂HPO₄ / 8 mM NaH₂PO₄ / 34 mM NaCl / 0.5% SDS) waschen. Anschließend die Membran 4 x 5 min mit 120 ml Puffer III (20 mM Tris-HCl / 100mM Diethanolamin / pH: 9.75!) äquilibrieren. Rocky: 12°/10 zum Einfüllen der Lösung, danach langsam auf 12°/6 zum Waschen/Äquilibrieren runterregulieren.
6. In der Zwischenzeit, 50µl CDP-Star (Tropix) zu 10 ml Puffer III (1:200) geben und gut mischen. Anschließend die Substratlösung in die Mitte der 'CDP-Star only'-Schale geben.
7. Membran nach dem letzten Äquilibrierungsschritt aus der Schale nehmen und kurz abtropfen lassen. Danach wird die Membran mit der DNA-Seite nach unten auf der Substratlösung plaziert. Durch mehrmaliges Aufnehmen und Ablegen der Membran wird sichergestellt, daß sich die Substratlösung gleichmäßig unter der Membran verteilt.
8. Membran kurz abtropfen lassen und danach auf einem Stück Plastik fixieren und mit Klarsichtfolie abdecken. 30 min. liegen lassen.
9. Röntgenfilm auflegen und für 30 min exponieren. Eventuell kürzere oder längere (maximal 2h) Expositionen durchführen.
10. Nach erfolgreicher Exposition die Membran wieder in die Schale überführen und mit 120 ml vorgewärmtem Puffer IV (5 mM EDTA / 1% SDS, 85°C) 6x 5 min strippen. Rocky: 12°/10 zum Einfüllen der Lösung, danach langsam auf 12°/4 zum Strippen runterregulieren.
11. Membran 1x mit 120 ml Puffer V (50 mM Tris-HCl / 150 mM NaCl) waschen und wieder bei Schritt 1 beginnen. Soll die Membran nicht sofort rehybridisiert werden so kann sie entweder in Puffer IV oder zwischen Klarsichtfolie zwischengelagert werden.

## Patentansprüche

1. Verfahren zur nicht-radioaktiven Detektion von Nukleinsäuren, welche DNA oder RNA oder Fragmente derselben darstellen, **dadurch gekennzeichnet, daß**. man Membran-gebundene DNA
a) mit einem speziellen Hybridisierungspuffer, bestehend aus Tris-HCl, Tris-Base, NaCl, Triton-X-100, SDS und Block Reagenz, bei Raumtemperatur vorinkubiert,
b) mit einer markierten Sonde in dem gleichen Puffer bei Raumtemperatur hybridisiert,
c) mehrmals mit einem Puffer I, bestehend aus P3S (pH ca. 7,3), SDS und Block Reagenz, wäscht,
d) mit Streptavidin-Alkalische-Phosphatase-Konjugat in Puffer I inkubiert,
e) einmal mit dem Puffer I, danach mehrmals mit einem Puffer II, bestehend aus PBS und SDS, wäscht, sowie mehrmals mit dem Puffer III, bestehend aus Tris-HCl und Diethanolamin, äquilibriert,
f) nach der Äquilibrierung die Membran nimmt und in eine Substratlösung, die sich aus CDP-Star (Tropix) und Puffer III zusammensetzt, überführt,
g) anschließend nach kurzem Abtropfen die Membran fixiert, abdeckt,
h) exponiert und
i) danach die Membran nimmt und mehrmals mit einem vorgewärmten Puffer IV, bestehend aus EDTA und SDS, strippt, danach einmal mit einem Puffer V, bestehend aus Tris-HCL und NaCl, wäscht und nach an sich bekannten Techniken auswertet.

2. Verfahren nach Anspruch 1,
**dadurch gekennzeichnet, daß** fünfmal mit Puffer I gewaschen wird.

3. Verfahren nach Anspruch 1 bis 2,
**dadurch gekennzeichnet, daß** sechsmal mit Puffer II gewaschen wird.

4. Verfahren nach Anspruch 1 bis 3,
**dadurch gekennzeichnet, daß** die Aquilibrierung bei pH 9,75 viermal mit Puffer III erfolgt.

5. Testkit zur nicht-radioaktiven Detektion von Membran-gebundenen Nukleinsäuren, welche DNA oder RNA oder Fragmente derselben darstellen, **dadurch gekennzeichnet,**
**daß** er besteht aus
a) einem Hybridisierungspuffer enthaltend 10xTNT, 20% SDS, Block Reagenz,
b) 10xPBS, enthaltend Na₂HPO₄, NaH₂PO₄, NaCl, pH: ca. 7.3,
c) einem Puffer I enthaltend Block Reagenz, 10xPBS, 20% SDS
d) einem Puffer II, enthaltend 10xPBS, 20% SDS,
e) 10xPuffer III enthaltend TrisHCl, Diethanolamin bei pH: 9.75,
f) einem Puffer IV enthaltend EDTA, 20% SDS
g) einem Puffer V enthaltend NaCl, TrisCl, pH8.0
h) 10xTNT enthaltend TrisHCl, TrisBase, NaCl, TritonX-100 pH: ca. 8.0.

6. Testkit nach Anspruch 5,
**dadurch gekennzeichnet, daß**
er besteht aus
a ) 5 l Hybridisierungspuffer enthaltend
500 ml 10xTNT
1250 ml 20% SDS
10g Block Reagenz
3250 ml H₂O bidest
b ) 5 l 10xPBS enthaltend
516.2 g Na₂HPO₄
132.6 g NaH₂PO₄
198.7 g NaCl
H₂O ad 5l
c ) 5 l Puffer I enthaltend
10 g Block Reagent
250 ml 10xPBS
125 ml 20% SDS
4625 ml H₂O bidest,
d ) 5 l Puffer II enthaltend
250 ml 10xPBS
125 ml 20% SDS
4625 ml H₂O bidest.
e ) 2 l 10xPuffer III enthaltend
64.4 g TrisHCl
1770 ml H₂O bidest
210.4 g Diethanolamin
f ) 5 l Puffer IV enthaltend
4700 ml H₂O bidest
50 ml 0.5M EDTA, pH8.0
250 ml 20% SDS
g ) 1 l Puffer V enthaltend
920 ml H₂O bidest
30 ml 5M NaCl
50 ml 1M TrisCl, pH8,
h ) 1 l 10xTNT enthaltend
44.4 g TrisHCl
25.6 g TrisBase
73 g NaCl
818 ml H₂O bidest
100 ml TritonX-100.

## Claims

1. Method for non-radioactive detection of nucleic acids portraying DNA or RNA of fragments thereof, wherein
a) membrane-bound DNA is pre-incubated at room temperature with a specific hybridisation buffer comprising Tris-HCl, Tris base, NaCl, Triton-X-100, SDS and block reagent,
b) is hybridised at room temperature in the same buffer with a marked probe,
c) is repeatedly washed with a buffer I, comprising PBS (pH approx. 7.3), SDS and block reagent,
d) is incubated with streptavidin/alkaline phosphatase conjugate in buffer I,
e) is washed once with buffer I, then repeatedly with a buffer II comprising PBS and SDS, and repeatedly equilibrated with buffer III, comprising Tris-HCl and diethanolamine,
f) the membrane is taken after the equilibration and transferred to a substrate solution composed of CDP-Star (Tropix) and buffer III,
g) then the membrane is fixed and covered after short dripping,
h) exposed and
i) then the membrane is taken and stripped with a preheated buffer IV comprising EDTA and SDS, then washed once with a buffer V, comprising Tris-HCl and NaCl, and evaluated according to techniques known per se.

2. Method according to Claim 1, wherein washing is done five times with buffer I.

3. Method according to Claims 1 to 2, wherein washing is done six times with buffer II.

4. Method according to Claims 1 to 3 wherein the equilibration is done four times with buffer III at pH 9.75.

5. Test-kit for non-radioactive detection of membrane-bound nucleic acids portraying DNA or RNA of fragments thereof, wherein it comprises
a) a hybridisation buffer containing 10xTNT, 20% SDS, block reagent,
b) 10xPBS, containing Na₂HPO₄, NaH₂PO₄, NaCl, pH: approx. 7.3,
c) a buffer I containing block reagent, 10xPBS, 20% SDS,
d) a buffer II containing 10xPBS, 20% SDS,
e) 10xbuffer III containing TrisHCl, diethanolamine at pH: 9.75
f) a buffer IV containing EDTA, 20% SDS
g) a buffer V containing NaCl, TrisCl, pH 8.0
h) 10 x TNT containing TrisHCl, TrisBase, NaCl, TritonX-100, pH: approx. 8.0

6. Test kit according to Claim 5 wherein it comprises
a) 5 l hybridisation buffer containing
500 ml 10 x TNT
1250 ml 20% SDS
10 g block reagent
3250 ml H₂O bidest
b) 5 l 10xPBS containing
516.2 g Na₂HPO₄
132.6 g NaH₂PO₄
198.7 g NaCl
H₂O ad 51
c) 5 l buffer I containing
10 g block reagent
250 ml 10xPBS
125 ml 20% SDS
4625 ml H₂O bidest,
d) 5 l buffer II containing
250 ml 10xPBS
125 ml 20% SDS
4625 ml H₂O bidest,
e) 2 l 10xbuffer III containing
64.4 g TrisHCl
1770 ml H₂O bidest,
210.4 g diethanolamine
f) 5 l buffer IV containing
4700 ml H₂O bidest,
50 ml 0.5M EDTA, pH 8.0
250 ml 20% SDS
g) 1 l buffer V containing
920 ml H₂O bidest,
30 ml 5M NaCl
50 ml 1M TrisCL, pH 8,
h) 1 l 10XTNT containing
44.4 g TrisHCl
25.6 g TrisBase
73 g NaCl
818 ml H₂O bidest,
100 ml TritonX-100.

## Revendications

1. Procédé de détection non radioactive d'acides nucléiques qui représentent l'ADN ou l'ARN ou des fragments de ces derniers, se caractérisant par le fait que l'on opère
a) à une incubation préliminaire à température ambiante avec un tampon d'hybridation spécial, composé de Tris-HCl, de Tris-base, de NaCl, de Triton X100, de SDS et d'un bloc réactif,
b) à une hybridation à température ambiante avec une sonde marquée dans le même tampon,
c) à plusieurs lavages avec un tampon I, composé de PBS (pH env. 7,3), SDS et d'un bloc réactif,
d) d'une incubation avec du conjugué streptavidine-phosphatase alcaline dans le tampon I,
e) à un lavage unique avec le tampon I, puis à plusieurs lavages avec un tampon II, composé de PBS et de SDS, ainsi qu'à plusieurs équilibrages avec le tampon III, composé de Tris-HCl et de diéthanolamine, de l'ADN lié à une membrane,
f) après l'équilibrage, à la prise de la membrane et à son transfert dans une solution de substrat qui se compose de CDP-Star (Tropix) et du tampon III,
g) ensuite à la fixation de la membrane après l'avoir fait un peu égoutter, à son recouvrement,
h) à son exposition et
i) à la prise de la membrane et à plusieurs lavages avec un tampon IV préchauffé, composé de EDTA et de SDS, au stripage, ensuite à un lavage avec un tampon V, composé de Tris-HCl et de NaCl et à l'évaluation selon des techniques connues en soi.

2. Procédé selon la revendication 1,
se caractérisant par le fait que la membrane soit lavée cinq fois avec le tampon I.

3. Procédé selon la revendication 1 à 2,
se caractérisant par le fait que la membrane soit lavée six fois avec le tampon II.

4. Procédé selon la revendication 1 à 3,
se caractérisant par le fait que l'équilibrage soit réalisé au pH 9,75 quatre fois avec le tampon III.

5. Kit test de détection non radioactive d'acides nucléiques liés à une membrane, qui représente l'ADN ou l'ARN ou des fragments de ces derniers et se caractérisant par le fait qu'il se compose de
a) un tampon d'hybridation contenant 10xTNT, SDS à 20 %, un bloc réactif
b) 10xPBS, comprenant Na₂HPO₄, NaH₂PO₄, NaCl, pH: env. 7,3.
c) un tampon I contenant un bloc réactif, 10xPBS, SDS à 20 %
d) un tampon II, contenant 10xPBS, SDS à 20 %,
e) 10x le tampon III, contenant du Tris-HCl, de la diéthanolamine au pH: 9,75
f) un tampon IV contenant de l'EDTA, SDS à 20 %
g) un tampon V contenant du NaCl, Tris-Cl, pH 8,0
h) 10x du TNT contenant du Tris-HCl, Tris base, NaCl, Triton X-100, pH: env. 8,0

6. Kit de test selon la revendication 5,
se caractérisant par le fait qu'il se compose de
a) 5 l de tampon d'hybridation contenant
500 ml de 10xTNT
1250 ml de SDS à 20 %
10 g de bloc réactif
3250 ml de H₂O bidist.
b) 5 l de 10xPBS contenant
516,2 g de Na₂HPO₄
132,6 g de NaH₂PO₄
198,7 g de NaCI
H₂O ad 5I
c) 5 l de tampon I contenant
10 g de bloc réactif
250 ml de 10xPBS
125 ml de SDS à 20 %
4625 ml de H₂O bidist.,
d) 5 l de tampon II contenant
250 ml de 10xPBS
125 ml de SDS à 20 %
4625 ml de H₂O bidist.,
e) 2 l de 10x tampon III contenant
64,4 g de Tris-HCl
1770 ml de H₂O bidist.
210,4 g de diéthanolamine
f) 5 l de tampon IV contenant
4700 ml de H₂O bidist.
50 ml de 0,5M EDTA, pH 8,0
250 ml de 20% de SDS
g) 1 l de tampon V contenant
920 ml de H₂O bidist.
30 ml de 5M NaCl
50 ml de 1M Tris-Cl, pH 8,
h) 1 l de 10xTNT contenant
44,4 g de Tris-HCl
25,6 g de Tris base
73 g de NaCl
818 ml de H₂O bidist.
100 ml de Triton X-100.
